# FASCICULE DE BREVET EUROPEEN

(11) **EP 1 393 236 B1**
(45) Date de publication et mention de la délivrance du brevet: **05.03.2008**
(21) Numéro de dépôt: 02761917.0
(22) Date de dépôt: 28.03.2002
(51) Int. Cl.: G06F 19/00, C12Q 1/68

(54) **PROCEDE D'ANALYSE DES LYMPHOCYTES T PAR LE BIAIS DES RECEPTEURS DES LYMPHOCYTES T D'UN ORGANISME**
VERFAHREN ZUR ANALYSE VON T-LYMPHOZYTEN ÜBER DIE T-LYMPHOZYTREZEPTOREN EINES ORGANISMUS
METHOD FOR ANALYZING T LYMPHOCYTES WITH THE AID OF T LYMPHOCYTE RECEPTORS OF AN ORGANISM

(30) Priorité: 13.04.2001 US 283378 P
(43) Date de publication de la demande: 03.03.2004
(73) Titulaire: INSTITUT NATIONAL DE LA SANTE ET DE LA RECHERCHE MEDICALE (INSERM), 75013 Paris (FR); CENTRE NATIONAL DE LA RECHERCHE SCIENTIFIQUE (CNRS), 75016 Paris (FR)
(72) Inventeur: SOULILLOU, Jean-Paul, F-44100 Nantes (FR); DELSUC, Marc-André, F-34000 Montpellier (FR); GUILLET, Marina, F-44130 BLAIN (FR); BROUARD, Sophie, F-44240 SUCE SUR ERDRE (FR)
(74) Mandataire: Ahner, Francis
(86) Numéro de dépôt international: PCT/FR2002/001087
(87) Numéro de publication internationale: WO 2002/084567

(56) Documents cités:
- GOROCHOV GUY ET AL: "Perturbation of CD4+ and CD8+ T-cell repertoires during progression to AIDS and regulation of the CD4+ repertoire during antiviral therapy." NATURE MEDICINE, vol. 4, no. 2, février 1998 (1998-02), pages 215-221, XP009005462 ISSN: 1078-8956 cité dans la demande
- PANNETIER CHRISTOPHE ET AL: "T-cell repertoire diversity and clonal expansions in normal and clinical samples." IMMUNOLOGY TODAY, vol. 16, no. 4, 1995, pages 176-181, XP002230780 ISSN: 0167-4919
- LANG R ET AL: "A rapid method for semiquantitative analysis of the human Vbeta-repertoire using TaqManPCR" JOURNAL OF IMMUNOLOGICAL METHODS, ELSEVIER SCIENCE PUBLISHERS B.V.,AMSTERDAM, NL, vol. 203, no. 2, 25 avril 1997 (1997-04-25), pages 181-192, XP004117430 ISSN: 0022-1759
- SEBILLE F ET AL: "Direct recognition of foreign MHC determinants by naive T cells mobilizes specific Vbeta families without skewing of the complementarity-determining region 3 length distribution." JOURNAL OF IMMUNOLOGY (BALTIMORE, MD.: 1950) UNITED STATES 15 SEP 2001, vol. 167, no. 6, 15 septembre 2001 (2001-09-15), pages 3082-3088, XP002230781 ISSN: 0022-1767
- GUILLET M ET AL: "TCR usage in naive and committed alloreactive cells: implications for the understanding of TCR biases in transplantation" CURRENT OPINION IN IMMUNOLOGY, CURRENT BIOLOGY LTD, XX, vol. 13, no. 5, 1 octobre 2001 (2001-10-01), pages 566-571, XP004304453 ISSN: 0952-7915

### LISTE DE SEQUENCES

<110> Institut National de la Santé et de la Recherche - INSERM
<120> Procédé d'analyse des récepteurs des lymphocytes T d'un organisme
<130> D19540
<150> US 06/283378
   <151> 2001-04-13
<160> 61
<170> PatentIn Ver. 2.1
<210> 1
   <211> 20
   <212> ADN
   <213> Rattus rattus
<400> 1
   tctccttccc attcacccac 20
<210> 2
   <211> 28
   <212> ADN
   <213> Rattus rattus
<400> 2
   tgactccacc caaggtctcc ttgtttga 28
<210> 3
   <211> 22
   <212> ADN
   <213> Rattus rattus
<400> 3
   gaatgcccag aaaagtccaa gt 22
<210> 4
   <211> 22
   <212> ADN
   <213> Rattus rattus
<400> 4
   ggcagaactc tgtactgcac ct 22
<210> 5
   <211> 22
   <212> ADN
   <213> Rattus rattus
<400> 5
   tcaaattcac cttggagcct ag 22
<210> 6
   <211> 23
   <212> ADN
   <213> Rattus rattus
<400> 6
   ctgagtgtcc cttaaactct ccc 23
<210> 7
   <211> 21
   <212> ADN
   <213> Rattus rattus
<400> 7
   aagtcgcttc caacctcaca a 21
<210> 8
   <211> 22
   <212> ADN
   <213> Rattus rattus
<400> 8
   tgagatgaac atgagtgcct tg 22
<210> 9
   <211> 23
   <212> ADN
   <213> Rattus rattus
<400> 9
   agaggcagat ggctgtttat ctc 23
<210> 10
   <211> 22
   <212> ADN
   <213> Rattus rattus
<400> 10
   ccctgattct ggagtctgct aa 22
<210> 11
   <211> 24
   <212> ADN
   <213> Rattus rattus
<400> 11
   cttctctctc attctggagt cagc 24
<210> 12
   <211> 18
   <212> ADN
   <213> Rattus rattus
<400> 12
   gagtcggctt ccccctct 18
<210> 13
   <211> 23
   <212> ADN
   <213> Rattus rattus
<400> 13
   tgctctctta acatcacctc tgc 23
<210> 14
   <211> 22
   <212> ADN
   <213> Rattus rattus
<400> 14
   agtcgcttct tgcctcagtc tt 22
<210> 15
   <211> 22
   <212> ADN
   <213> Rattus rattus
<400> 15
   tgaagatcca gaccacacaa cc 22
<210> 16
   <211> 20
   <212> ADN
   <213> Rattus rattus
<400> 16
   tcatccatct ccacgctgaa 20
<210> 17
   <211> 24
   <212> ADN
   <213> Rattus rattus
<400> 17
   cagagtgact cagccaccta tctc 24
<210> 18
   <211> 24
   <212> ADN
   <213> Rattus rattus
<400> 18
   tcactcactc tggcttctac ctct 24
<210> 19
   <211> 22
   <212> ADN
   <213> Rattus rattus
<400> 19
   cacatcctga tgacagaggc tt 22
<210> 20
   <211> 22
   <212> ADN
   <213> Rattus rattus
<400> 20
   ctctgaagat ccaacgcaca ac 22
<210> 21
   <211> 23
   <212> ADN
   <213> Rattus rattus
<400> 21
   gctcagtgtg tcccagactt aca 23
<210> 22
   <211> 21
   <212> ADN
   <213> Rattus rattus
<400> 22
   aacgcaagac ctgaagacag c 21
<210> 23
   <211> 24
   <212> ADN
   <213> Rattus rattus
<400> 23
   agatcaagtc cagcaagcta acag 24
<210> 24
   <211> 21
   <212> ADN
   <213> Rattus rattus
<400> 24
   cactgtacct gtgctccagc a 21
<210> 25
   <211> 24
   <212> ADN
   <213> Rattus rattus
<400> 25
   cactgatgtt ctgtgtgaca ggtt 24
<210> 26
   <211> 20
   <212> ADN
   <213> Rattus rattus
<400> 26
   gtgaatggga aggagatccg 20
<210> 27
   <211> 25
   <212> ADN
   <213> Rattus rattus
<400> 27
   ttctggcaca atcctcgcaa ccact 25
<210> 28
   <211> 27
   <212> ADN
   <213> Homo sapiens
<400> 28
   tggcctccca tctccttcat cacatct 27
<210> 29
   <211> 21
   <212> ADN
   <213> Homo sapiens
<400> 29
   ggacaggact gaacgtcttg c 21
<210> 30
   <211> 21
   <212> ADN
   <213> Homo sapiens
<400> 30
   ttgagcacac agagggctac a 21
<210> 31
   <211> 22
   <212> ADN
   <213> Homo sapiens
<400> 31
   tactgcctga gcagccgcct ga 22
<210> 32
   <211> 19
   <212> ADN
   <213> Homo sapiens
<400> 32
   ggtcgctgtg tttgagcca 19
<210> 33
   <211> 20
   <212> ADN
   <213> Homo sapiens
<400> 33
   tcctgggtcc actcgtcatt 20
<210> 34
   <211> 21
   <212> ADN
   <213> Homo sapiens
<400> 34
   tgttcccacc cgaggtcgct g 21
<210> 35
   <211> 22
   <212> ADN
   <213> Homo sapiens
<400> 35
   gatctctgct tctgatggct ca 22
<210> 36
   <211> 22
   <212> ADN
   <213> Homo sapiens
<400> 36
   ttccctgact tgcactctga ac 22
<210> 37
   <211> 22
   <212> ADN
   <213> Homo sapiens
<400> 37
   cctgaccttg tccactctga ca 22
<210> 38
   <211> 22
   <212> ADN
   <213> Homo sapiens
<400> 38
   ccagcaccaa ccagacatct at 22
<210> 39
   <211> 20
   <212> ADN
   <213> Homo sapiens
<400> 39
   taccgtcagc aacctggaca 20
<210> 40
   <211> 20
   <212> ADN
   <213> Homo sapiens
<400> 40
   gggccttcag ttcctctttg 20
<210> 41
   <211> 20
   <212> ADN
   <213> Homo sapiens
<400> 41
   agctgaatgt gaacgccttg 20
<210> 42
   <211> 22
   <212> ADN
   <213> Homo sapiens
<400> 42
   gatcacacag gtgctggagt ct 22
<210> 43
   <211> 22
   <212> ADN
   <213> Homo sapiens
<400> 43
   atccaatttc tgaacacaac cg 22
<210> 44
   <211> 23
   <212> ADN
   <213> Homo sapiens
<400> 44
   gatcacacag gagctggagt ctc 23
<210> 45
   <211> 22
   <212> ADN
   <213> Homo sapiens
<400> 45
   caagtcgctt ctcacctgaa tg 22
<210> 46
   <211> 24
   <212> ADN
   <213> Homo sapiens
<400> 46
   ttctggtaca gacagaccat gatg 24
<210> 47
   <211> 22
   <212> ADN
   <213> Homo sapiens
<400> 47
   tcttcacatc aattccctgg ag 22
<210> 48
   <211> 22
   <212> ADN
   <233> Homo sapiens
<400> 48
   cctctcagta cctctgtgcc ag 22
<210> 49
   <211> 21
   <212> ADN
   <213> Homo sapiens
<400> 49
   gcccaagaca caaggtcaca g 21
<210> 50
   <211> 19
   <212> ADN
   <213> Homo sapiens
<400> 50
   agacctggga catgggctg 19
<210> 51
   <211> 19
   <212> ADN
   <213> Homo sapiens
<400> 51
   tcgacaagac ccaggcatg 19
<210> 52
   <211> 25
   <212> ADN
   <213> Homo sapiens
<400> 52
   gatggttata gtgtctccag agcaa 25
<210> 53
   <211> 22
   <212> ADN
   <213> Homo sapiens
<400> 53
   agggagatgt tcctgaaggg ta 22
<210> 54
   <211> 25
   <212> ADN
   <213> Homo sapiens
<400> 54
   tctgatggat acagtgtctc tcgac 25
<210> 55
   <211> 22
   <212> ADN
   <213> Homo sapiens
<400> 55
   tctactctga aggtgcagcc tg 22
<210> 56
   <211> 22
   <212> ADN
   <213> Homo sapiens
<400> 56
   tttcctctca ctgtgacatc gg 22
<210> 57
   <211> 22
   <212> ADN
   <213> Homo sapiens
<400> 57
   cttatttctg tgccagctca cc 22
<210> 58
   <211> 22
   <212> ADN
   <213> Homo sapiens
<400> 58
   ctccactctc aagatccagc et 22
<210> 59
   <211> 21
   <212> ADN
   <213> Homo sapiens
<400> 59
   ccacaaagct ggaggactca g 21
<210> 60
   <211> 22
   <212> ADN
   <213> Homo sapiens
<400> 60
   cccagttcct catttcgttt ta 22
<210> 61
   <211> 22
   <212> ADN
   <213> Homo sapiens
<400> 61
   ctttctgctt tcttgacatc cg 22

## Description

L'invention concerne l'étude du système immunitaire des organismes tels que les hommes ou les animaux, en particulier l'analyse des lymphocytes T activés ou non d'un organisme par l'intermédiaire de l'étude de son récepteur.

Les réponses immunitaires peuvent être classées en deux grandes catégories: les réponses naturelles et les réponses adaptatives (ou spécifiques). Les cellules impliquées dans la réponse naturelle utilisent des systèmes de reconnaissance primitifs et non spécifiques. Ce type de réponse représente donc la première ligne de défense contre les infections et prédomine aux stades initiaux de l'infection. Dans le cas où l'immunité naturelle est inefficace, l'immunité adaptative, qui met en jeu des réponses spécifiques adaptées à chaque micro-organisme, se met en place. Les lymphocytes sont à l'origine de cette reconnaissance adaptative.

Il existe deux types de lymphocytes: les lymphocytes B, responsables de l'immunité humorale (production d'anticorps) et les lymphocytes T, responsables de l'immunité cellulaire. Ces derniers jouent un rôle central en biologie. Ils reconnaissent des peptides issus de la dégradation intracellulaire d'antigènes mais seulement quand ces peptides sont associés à des molécules portées par les cellules présentatrices d'antigène appelés molécules du Complexe Majeur d'Histocompatibilité ou CMH (aussi appelé Human Leucocyte Antigen ou HLA), portées par les cellules présentatrices d'antigène. Pour cette fonction, les lymphocytes T utilisent un récepteur spécifique appelé récepteur d'antigène des lymphocytes T ou TCR.

Le récepteur des cellules T (TCR) est un hétérodimère constitué de deux chaînes : α et β. Nous nous intéressons ici uniquement à la chaîne β qui comprend les gènes Vβ (24 chez l'homme), Dβ (2 chez l'homme), Jβ (12 chez l'homme) et Cβ (2 chez l'homme), initialement séparés sur l'ADN. Un des gènes Vβ se lie de façon aléatoire à l'un des gènes Dβ, Jβ et Cβ pour former un récepteur fonctionnel. Ainsi, un grand nombre de TCR différents peut être théoriquement généré (environ 25.10⁶ TCR différents). La rencontre d'un antigène entraîne la sélection de lymphocytes T portant un TCR spécifique constitué d'un assemblage Vβ-Dβ-Jβ particulier. La reconnaissance de l'antigène se fait principalement au niveau de la région hypervariable CDR3 (Complementary Determining Region 3) qui est formée par le réarrangement des gènes Vβ, Dβ et Jβ et qui est de longueur variable. Normalement, chez un individu sain, les transcrits codant pour une famille Vβ quelconque utilisent toutes les longueurs possibles du CDR3.

Jusqu'à maintenant, l'analyse du récepteur des cellules T a principalement été réalisée de manière "qualitative" (i.e.: recherche d'une éventuelle sélection) grâce à des méthodes telles que celle mise en oeuvre par le logiciel connu sous la marque Immunoscope^{®} et qui étudie ce récepteur au niveau de la distribution des longueurs de la région CDR3, région qui serait la plus fortement impliquée dans la reconnaissance du peptide.

Après amplification de cette région CDR3, de longueur variable, grâce à une amorce Cβ et une amorce Vβ, le logiciel Immunoscope fournit les résultats sous forme de pics, chaque profil étant constitué de 7 à 11 pics, c'est-à-dire de 7 à 11 tailles différentes du CDR3, séparées chacune de 3 nucléotides. Ainsi, lorsque toutes les longueurs du CDR3 sont représentées dans une famille Vβ donnée, on observe un profil gaussien, caractéristique d'une réponse polyclonale. C'est par exemple ce que l'on observe, en général, chez un individu sain. A l'inverse, une longueur du CDR3 peut être majoritairement représentée à la suite d'une activation particulière ayant sélectionné des clones de lymphocytes T particuliers et on observe alors une "altération" oligo- ou monoclonale de la distribution des longueurs de la région CDR3, les profils perdant alors leur caractère gaussien.

Un moyen de comparer le profil d'un échantillon par rapport au profil témoin est d'utiliser le calcul décrit dans l'article de G. Gorochov (Nat. Med., 1998, 4(2) :215) qui calcule la différence entre l'aire sous chaque pic du profil témoin et l'aire sous chaque pic du profil de l'échantillon. On obtient ainsi des pourcentages d'altération par rapport au gaussien et les résultats sont représentés sous forme de structures tridimensionnelles qui permettent de visualiser les altérations associées à toutes les familles Vβ d'un échantillon donné sur un même graphique. Ces graphiques, communément appelés Reperturb, apparaissent plans lorsque le répertoire n'est pas altéré et perturbés lorsque le répertoire est altéré.

Malgré leur intérêt, ces méthodes d'analyse qualitative du récepteur des cellules T ne permettent pas d'avoir une connaissance précise de la quantité de mRNA Vβ affectée par un processus de sélection (et donc indirectement de l'importance du pool de cellules T utilisant un réarrangement Vβ donné) et présentent donc une limite importante en étant incapables de hiérarchiser les anomalies constatées.

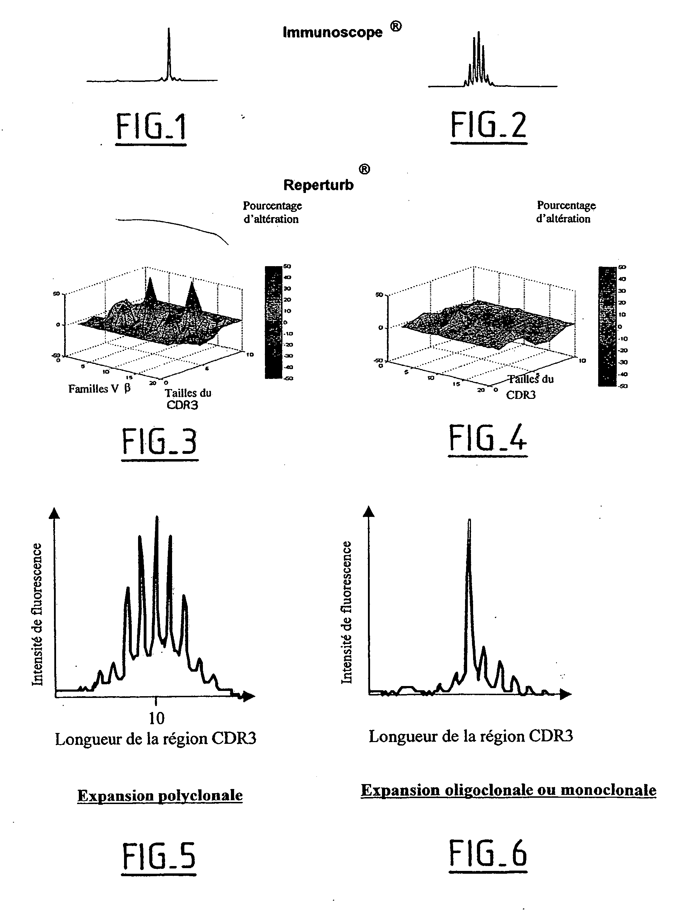

A titre d'exemple, la figure 2 représente un profil gaussien et la figure 1 une expansion monoclonale obtenus par Immunoscope^{®}. En utilisant la méthode Reperturb, quatre pics correspondant chacun à une famille Vβ présentant une expansion clonale, apparaissent sur la figure 3. Malgré son intérêt, cette analyse qualitative seule ne permet pas de savoir si les TCR présentant ces expansions intéressent une fraction importante du transcriptome Vβ ou s'ils ne représentent qu'une minorité parmi le pool de cellules T. Par exemple, sur la figure 3, on ne sait pas si les cellules T portant l'une des 4 expansions clonales est plus fortement représentée qu'une autre parmi la population de cellules T totales et est donc plus particulièrement impliquée dans la réaction immunitaire étudiée. A l'inverse, le graphique reste plat quand toutes les familles Vβ présentent une distribution gaussienne des longueurs du CDR3 comme le montre la figure 4. Ce profil plat peut être dû :
- à une absence de réponse (aucun lymphocyte T n'est impliqué)
- à une réponse polyclonale due à l'activation d'un grand nombre de clones T, cette activation masquant la présence d'expansions clonales potentielles
- ou à l'activation de cellules T sans modification de la distribution de longueur du CDR3. C'est par exemple le cas d'un profil observé après stimulation par des ligands polyclonaux (anticorps anti-CD3, Concanavaline A, PHA, etc...) ou par des superantigènes.

L'analyse qualitative seule ne permet pas de différencier entre ces trois hypothèses.

Le demandeur a été le premier à utiliser la méthode Immunoscope^{®} pour étudier le rejet et la tolérance d'allogreffes ou, plus récemment, de xénogreffes. Cependant, bien que ces études aient été réalisées sur des lignées de rats congéniques (une combinaison beaucoup plus "simplifiée" que ne l'est la combinaison allogénique), elles ont révélé des altérations de type privé (spécifiques d'un individu) pour la plupart tandis que les altérations publiques (même longueur et même séquence du CDR3 retrouvée chez plusieurs animaux) sont des événements extrêmement rares. Du fait de ce profil privé dominant (et donc apparemment chaotique) et de la forte réactivité croisée du TCR, aucune information reproductible sur la physiologie de l'alloreconnaissance ne peut être obtenue. En partant de ces observations et de ce constat, il apparaît qu'une analyse combinée des altérations qualitatives (privées / publiques) et de l'estimation quantitative du pool de cellules T intervenant dans ces altérations (nombre de transcrits Vβ pour chaque profil altéré) est le seul moyen d'approcher la cinétique de la réponse des lymphocytes T.

Ainsi, notre capacité d'évaluation et d'étude de la dynamique et de l'importance de la réponse des lymphocytes T in vivo est très limitée. L'analyse du répertoire du TCR est un reflet indirect de l'état de la mobilisation et de l'activation des cellules T dans une situation biologique donnée. Cependant, l'importante réactivité croisée du TCR, ainsi que la grande diversité des peptides apprêtés, rend difficile l'utilisation des altérations qualitatives du répertoire Vβ prise isolément (par exemple suivant la méthode Immunoscope) en tant que marqueur pour identifier et hiérarchiser de façon précise la population de cellules T impliquée dans une réaction immunitaire complexe même si l'étude du répertoire se fait au niveau des séquences et des variations de longueur de la région CDR3. Malgré leur intérêt, les méthodes préalablement connues d'analyse du TCR ne permettaient donc pas d'avoir une connaissance précise du pool de mRNA de lymphocytes T (par rapport au transcriptome Cβ global) utilisant un réarrangement des chaînes Vβ données. En outre, l'accumulation des transcrits Vβ est un paramètre différent de l'expression des protéines correspondantes à la surface des cellules T car ces dernières peuvent être sous-régulées après interaction avec l'antigène.

Un des buts de l'invention est de fournir une nouvelle méthode d'analyse qui permette d'ajouter une dimension quantitative, paramètre essentiel à l'analyse des altérations du répertoire.

En vue de la réalisation de ce but, nous proposons, selon l'invention, un procédé d'analyse des récepteurs des lymphocytes T d'un organisme, qui comprend les étapes consistant à :
- déterminer le nombre de transcrits pour chaque gène Vβ, pour obtenir des premières données. Ce nombre de transcrits peut ensuite être représenté par exemple sous forme de valeurs brutes (nombre de transcrits pour chaque famille Vβ), sous forme de ratio entre ce nombre de transcrits et le nombre de transcrits d'un gène référence, par exemple le gène HPRT (hypoxanthine phosphoribosyl transférase), ce dernier étant un gène présent dans toutes les cellules et qui permet d'uniformiser les données, sous forme de pourcentage de chaque transcrit Vβ parmi la somme des transcrits Vβ ou sous forme d'une mesure de la variation entre le nombre de transcrits dans une famille Vβ donnée d'un échantillon donné et le nombre de transcrits de la même famille Vβ d'un échantillon de référence.
- amplifier la région CDR3 des récepteurs des lymphocytes T pour chacun des gènes Vβ associés à l'organisme,
- séparer les différentes longueurs du CDR3 des récepteurs des lymphocytes T pour chacun des gènes Vβ associé à l'organisme et estimer la proportion des transcrits de chaque longueur du CDR3 dans chaque famille Vβ pour obtenir des deuxièmes données,
- représenter dans un diagramme à quatre variables les premières et deuxièmes données en fonction des gènes Vβ et de la longueur des régions CDR3.

Ainsi, l'invention permet de suivre précisément l'existence et la hiérarchie quantitative d'anomalies de la distribution des longueurs de la région CDR3 au cours de l'initiation, la cinétique, l'expansion, la mémoire et le degré d'épitope spreading dans une situation de réponse immune complexe (par exemple un contexte pathologique), en raison du fait que les deux paramètres (profil d'altération de la distribution des longueurs de la région CDR3 du TCR par rapport à la situation de repos et évaluation quantitative des transcrits Vβ du pool de cellules T impliquées) peuvent être globalement visualisés, de façon simultanée, sur le diagramme.

L'invention offre donc une vue intégrée des altérations du TCR, au lieu de la seule description individuelle des altérations de chaque famille Vβ. Elle est plus propice à la compréhension des événements immunologiques fondamentaux et appliqués impliquant une réponse lymphocytaire T (maladies infectieuses, maladies auto-immunes, cancers, greffes, etc). La présentation des résultats est aussi à la fois complète, didactique et facile à appréhender.

L'invention propose une nouvelle stratégie d'évaluation de la réponse immune in vivo, de préférence fondée sur une intégration assistée par ordinateur des altérations du transcriptome Vβ des lymphocytes Tαβ activés.

Le procédé de l'invention combine l'analyse obtenue par exemple par les programmes Reperturb et Immunoscope^{®} de l'altération de la longueur du CDR3 (dans une famille Vβ donnée), comparée avec le profil gaussien au repos, avec une mesure quantitative de chaque transcrit Vβ par PCR quantitative (par exemple suivant la méthode connue sous le nom de TaqMan) de chaque famille Vβ et donc des transcrits pour tous les signaux TCR altérés. Une analyse factorielle est utilisée pour définir les cohérences d'utilisation des Vβ et de leur altération. L'invention inclut un programme d'expression graphique permettant une vue générale et par exemple tridimensionnelle de la hiérarchie des altérations et donc de l'état d'activation des cellules T réactives.

Le procédé selon l'invention pourra présenter en outre au moins l'une quelconque des caractéristiques suivantes :
- au moins une des variables est représentée sans être associée à une dimension de l'espace ;
- au moins une des variables est représentée par des différences d'aspect local du diagramme;
- les différences d'aspect local sont des différences de couleur ;
- la variable qui n'est pas associée à une dimension de l'espace correspond aux deuxièmes données ;
- les premières données sont représentées suivant la direction verticale ;
- les valeurs prédéterminées correspondent, pour chaque gène Vβ, à une répartition gaussienne des proportions de transcrits correspondant aux régions. CDR3 associées à ce gène ;
- il comprend, après la détermination des premières et des deuxièmes données, les étapes consistant à choisir un gène Vβ en fonction des résultats des deux premières étapes, et à extraire de la population de cellules T totale les cellules T portant un TCR constitué par cette famille Vβ préalablement choisie ; et
- au moins certaines des étapes sont mises en oeuvre de façon automatisée.

On prévoit un programme informatique pour l'analyse des récepteurs des lymphocytes T d'un organisme, apte à commander la mise en oeuvre d'au moins l'étape de représentation du procédé selon l'invention.

On prévoit en outre selon l'invention une installation d'analyse des récepteurs des lymphocytes T d'un organisme, comprenant:
- des moyens automatisés pour mesurer les quantités de chaque gène Vβ et éventuellement le rapport de ces quantités à des valeurs prédéterminées pour obtenir des premières données ;
- des moyens automatisés pour amplifier la région CDR3 pour chacun des gènes Vβ des récepteurs des lymphocytes T associés à l'organisme, et pour mesurer, pour chaque gène Vβ, la quantité relative de chaque région CDR3 par rapport à la totalité des régions CDR3 associées à un gène Vβ pour obtenir des deuxièmes données ; et
- des moyens automatisés pour représenter dans un diagramme à quatre variables les premières et deuxièmes données en fonction des gènes Vβ et de la longueur des régions CDR3. Ce diagramme permet une vision simplifiée et globale du processus complexe de mobilisation des cellules T au cours d'une réaction immunitaire.

On prévoit un support d'information présentant un diagramme d'analyse des récepteurs des lymphocytes T d'un organisme, comprenant les quatre variables suivantes :
- les gènes Vβ associés à l'organisme ;
- la longueur des régions CDR3 des récepteurs des lymphocytes T ;
- des premières valeurs représentant la quantité de transcrits de chaque gène Vβ sous forme de quantité relative ou absolue ;
- des deuxièmes données représentant pour chacune des longueurs de la région CDR3 dans chaque gène Vβ la différence entre la proportion des transcrits correspondants à une région CDR3 d'un gène Vβ dans un échantillon donné par rapport aux transcrits correspondant à la même région CDR3 du même gène Vβ d'un échantillon de référence qui est un échantillon présentant une distribution gaussienne des longueurs du CDR3.

L'invention peut faire l'objet de nombreuses applications, par exemple dans les secteurs immunologiques suivants :
- suivi de l'évolution de différentes pathologies au cours du temps telles que les cancers, les maladies virales (SIDA, infection à BBV...), les maladies auto-immunes...;
- suivi de l'effet de différents traitements (immunothérapie...);
- étude des mécanismes de la réponse immunitaire dans différentes maladies et/ou suite à différentes thérapies ;
- étude des mécanismes de rejet et de tolérance en transplantation ;
- diagnostique d'un « état mémoire » ;

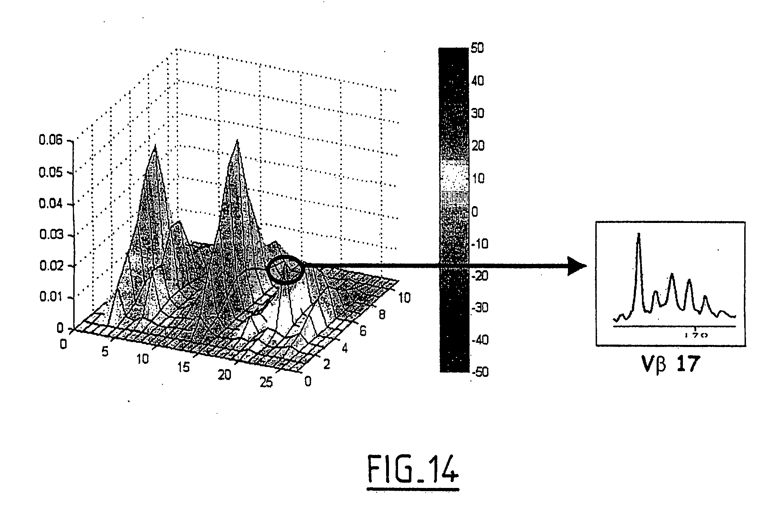

Nous proposons ci-après plus précisément quelques exemples d'applications:
- suivi de l'évolution de la maladie et recherche d'une éventuelle corrélation entre la régression ou l'aggravation de la maladie et l'importance du "spreading", qui correspond au fait que la sélection des clones T va évoluer au cours du temps du fait de la reconnaissance de nouvelles structures antigéniques par mimétisme moléculaire
- suivi de l'évolution, dans le temps, du répertoire des cellules T chez des patients présentant, ou non, une régression continue de la maladie après immunothérapie. L'invention permettra à la fois de suivre l'effet de la thérapie et le développement de la réponse immune contre la maladie;
- détection de marqueurs spécifiques liés à l'évolution de la maladie. Par exemple, une augmentation des altérations clonales, une augmentation du nombre de familles Vβ présentant des altérations particulières et/ou une augmentation significative des messagers Vβ dans le sang prélevé selon des intervalles de temps importants, pourra permettre d'étudier l'"epitope spreading". De telles différences permettront discriminer les réponses T avant et après un traitement des réponses plus fortement impliquées lors de la régression de la maladie due à la thérapie ;
- distinction entre les processus immuns précoces de ceux mis en place depuis plus longtemps dans le cas de maladies auto-immunes par exemple et entre les processus immuns impliqués dans différentes formes d'une même maladie ;
- comparaison des représentations graphiques fournies par l'invention avec les résultats obtenus par des techniques classiques de diagnostique et d'analyse immunologique réalisées par d'autres méthodes (par exemple : suivi des cellules T par tétramères, suivi de la production des cytokines par la méthode connue sous le nom Elispot) et possibilité de corréler l'évolution de l'apparition et de la disparition de clones T au cours du temps avec l'évolution clinique du patient pour obtenir des informations importantes sur la sémiologie et les processus immuns impliqués dans la maladie.
- Différentes modifications pourront être observées après traitement d'une maladie (disparitions de clones, modification des valeurs quantitatives, modification de la topologie globale des profils...). Ces modifications peuvent indiquer une sous-régulation, la délétion de clones auto-réactifs ou encore l'évolution des cellules T impliquées. Cette sémiologie peut varier suivant la stratégie utilisée. En effet, certains agents peuvent induire l'apoptose des cellules auto-réactives alors que les drogues cytotoxiques telles que la cyclosporine ou la mitoxantrone peuvent entraîner des modifications aspécifiques. Théoriquement, les profils des patients obtenus grâce à l'invention pourraient donc influencer la décision du traitement. L'invention peut également être utile pour l'établissement de critères de tolérance chez des patients apparemment dépourvus de symptômes cliniques ;
- possibilité d'identifier, au niveau périphérique, des représentations spécifiques de certaines maladies. De même, on envisage la possibilité de superposer les graphiques obtenus à partir du sang et ceux observés au sein du greffon (par exemple pour la détection de signes de rejet chronique via une prise de sang);
- L'analyse selon l'invention sur des prélèvements de patients pourra permettre de définir un nouveau paramètre de sélection d'épitopes candidats à une immunothérapie (antitumorale par exemple) plus efficace.
- isolement de cellules T mises en évidence par la combinaison des premières et des deuxièmes données comme étant particulièrement impliquées dans la réaction immunitaire étudiée. Une analyse de la spécificité et de la fonctionnalité de ces cellules T isolées pourra ensuite être réalisée. Cette identification puis isolement de cellules T portant un TCR particulier permet de réaliser des études fonctionnelles ou phénotypiques par exemple non pas sur la population de cellules T totales mais uniquement sur les cellules T impliquées dans la réaction immunitaire étudiée. Cette démarche de "gene searching" améliorée permettra d'identifier de nouvelles stratégies d'immunothérapies. A titre d'exemple, voir la figure 14.

L'enjeu de nombreuses procédures vaccinantes est de développer une réponse T et donc de la mesurer. L'invention représente à ce titre un apport nouveau potentiellement très utile. En outre, l'étude de la topologie des répertoires représentés peut être importante pour déceler les "signatures" spécifiques d'un processus pathologique chez un individu. En effet, l'invention peut être utilisée, par exemple, pour analyser l'évolution du processus pathologique chez des patients et/ou pour influencer la décision d'un traitement.

Dans le domaine de la recherche pharmaceutique et médicale, l'invention servira pour la compréhension et le suivi de pathologies et/ou de traitements dans différents domaines tels que:
- virologie ;
- cancérologie ;
- maladies auto-immunes ;
- traitement des allergies (suivi des désensibilisations) ;
- transplantation d'organes, de tissus ou de moelle, anticipation de la survenue d'un rejet aigu ou chronique, recherche de clones régulateurs chez des patients greffés afin de savoir si ceux-ci sont susceptibles d'être tolérants à leur greffon en l'absence de tout traitement immunosuppresseur....

En recherche médicale, l'invention servira par exemple comme outil clinique pour l'aide au diagnostique (maladies virales, maladies auto-immunes, cancer) et le suivi des thérapies (vaccins, thérapies cellulaires, thérapies géniques).

L'invention peut être utilisée sur l'animal dans le cadre de projets de recherche (tests animaux, analyses précliniques,...), et sur l'homme dans le cadre d'évaluation de traitements.

D'autres caractéristiques et avantages de l'invention apparaîtront encore dans la description suivante de plusieurs modes préférés de réalisation donnés à titre d'exemples non-limitatifs en référence aux dessins annexés :

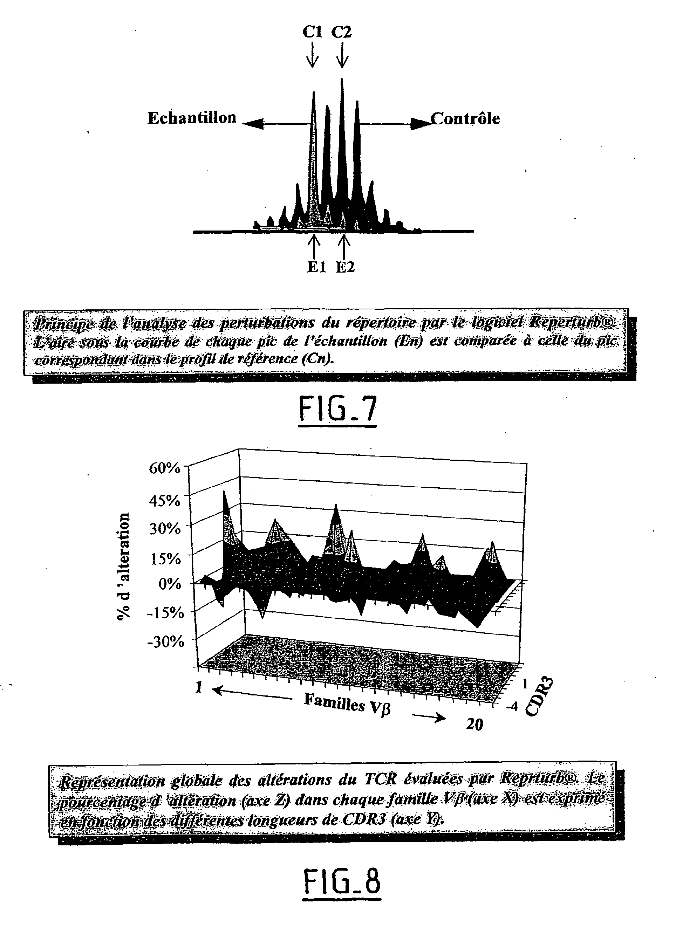

- les figures 1 à 8 sont des diagrammes relatifs à l'étude des lymphocytes T et obtenus avec des techniques de l'art antérieur ;
- les figures 9, 11 à 13 sont des diagrammes obtenus avec le procédé selon l'invention ;
- la figure 10 est une représentation schématique des moyens de mise en oeuvre de l'invention et du support obtenu ; et
- la figure 14 représente un exemple de "sorting". Dans cet exemple, un profil TcLandscape a été obtenu à partir d'un patient attaeint de sclérose en plaque. Sur le graphique, on s'aperçoit que la famille Vβ17 est altérée et que le nombre de transcrits Vβ17 est important. Les cellules T portant un TCR composé des gènes Vβ17 ont été isolées par cytométrie de flux et des études fonctionelles ont été réalisées sur ces cellules isolées. On observe alors une transcription particulièrement élevée des cytokines I1-6, I1-8 et TNFa dans ces cellules. Cette accumulation n'est pas observée dans les cellules T portant un TCR Vβ17+ d'un sujet sain. D'autre part, ces cellules Vβ17+ isolées à partir du patient présentent un phénotype activé.

### EXEMPLES

Nous allons décrire un premier mode de mise en oeuvre de la méthode selon l'invention dans le contexte de la réponse cellulaire en xénotransplantation.

En transplantation, les cellules T peuvent reconnaître les peptides antigéniques présentés par les cellules présentatrices du receveur (voie indirecte) ou du donneur (voie directe). La voie de présentation directe étant particulièrement impliquée au cours des rejets aigus, les inventeurs ont étudié l'impact de ce type de reconnaissance sur le répertoire du TCRβ. Grâce à l'invention, qui permet d'ajouter une dimension quantitative aux altérations qualitatives de la distribution des longueurs de la région CDR3, il est apparu que cette distribution n'était pas altérée dans la reconnaissance directe alors que les cellules T sont sélectionnées en fonction de leur segment Vβ. De plus, en suivant la régulation de l'expression du TCR comme marqueur d'activation, on montre qu'une proportion importante des cellules T naïves peuvent reconnaître des cellules présentatrices « étrangères », phénomène qui permet de mieux comprendre l'ampleur de la réponse directe et son implication dans le rejet aigu.

L'analyse du répertoire du TCR permet d'évaluer la diversité d'une population de lymphocytes T et d'en suivre l'évolution dans différentes conditions expérimentales ou au cours de la progression d'une pathologie donnée. On peut ainsi mettre en évidence des restrictions (absence de certaines familles Vβ) ou des altérations du répertoire (modification de la répartition des longueurs de CDR3 dans une famille Vβ).

L'analyse du répertoire du TCR permet d'évaluer la diversité d'une population de lymphocytes T et d'en suivre l'évolution dans différentes conditions expérimentales ou au cours de la progression d'une pathologie donnée. On peut ainsi mettre en évidence des restrictions (absence de certaines familles Vβ) ou des altérations du répertoire (modification de la répartition des longueurs de CDR3 dans une famille Vβ).

Différentes techniques ont été développées afin d'analyser la région CDR3 et/ou de quantifier les variations du répertoire au niveau de la chaîne Vβ du TCR. Actuellement, l'analyse quantitative du TCR peut être réalisée par différentes méthodes: la RNAse Protection Assay qui présente le désavantage d'être difficile techniquement, la quantification à partir d'une PCR compétitive sur CD3, qui ne discrimine pas les différentes familles Vβ, les anticorps monoclonaux anti-Vβ. Pour l'analyse quantitative, de nombreux anticorps monoclonaux dirigés contre les différentes régions variables du TCR αβ sont disponibles chez l'homme et chez la souris et permettent une analyse de l'expression au niveau protéique. Cependant, chez le rat, seul trois anticorps sont disponibles et efficaces, ce qui limite l'intérêt de cette méthode. De plus, l'analyse uniquement au niveau protéique ne rend pas compte des événements de sélection se produisant au niveau de la région CDR3, qui est à la base de la spécificité de la reconnaissance de l'antigène. De plus, le pourcentage d'expression d'une protéine Vβ n'est pas représentatif du pourcentage de transcrits Vβ correspondant. En effet, une telle quantification par des anticorps risque de sous-estimer le nombre de TCR car ceux-ci sont internalisés après activation. D'autres techniques, basées sur l'amplification par PCR des régions variables du TCR à l'aide d'amorces spécifiques, permettent d'analyser le répertoire à partir d'ADN génomique. Les produits d'amplification ainsi obtenus peuvent être analysés par autoradiographie, clonés et séquencés.

L'analyse qualitative de la chaîne β du TCR (deuxièmes données) est basée sur l'étude de la distribution des longueurs de la région hypervariable CDR3 du TCR dans chaque famille Vβ d'une population de lymphocytes T donnée. Un pool de cellules T au repos est caractérisé par une répartition gaussienne des différentes longueurs du CDR3 dans chaque famille Vβ. La mobilisation de clones T spécifiques suite à la reconnaissance d'un antigène s'accompagne de l'usage préférentiel de certains TCR composés d'une famille Vβ et d'une longueur du CDR3 particulière.

Le principe de la technique Immunoscope® consiste en une amplification, par PCR, des différentes régions CDR3 en utilisant une amorce 3' commune placée dans le segment Cβ et une amorce 5' spécifique de chaque famille Vβ (voir liste des séquences). Les produits amplifiés sont ensuite soumis à une étape d'élongation effectuée avec une seconde amorce Cβ marquée par un fluorophore. Les produits d'amplification fluorescents sont séparés en fonction de leur longueur par migration sur gel de polyacrylamide. Le profil de migration est analysé par un séquenceur d'ADN couplé au logiciel Immunoscope®. On obtient ainsi en référence à la figure 5 une image de la distribution des longueurs CDR3 pour chaque couple d'amorces. On peut observer entre 1 et 11 pics d'amplification correspondant chacun à une taille particulière de la région CDR3 et séparés chacun de trois nucléotides. Cette analyse peut être encore affinée si on utilise une amorce spécifique d'un segment Jβ au lieu de l'amorce commune Cβ. Comme illustré à la figure 6, l'analyse de la répartition des différentes longueurs de CDR3 permet d'identifier d'éventuelles expansions oligoclonales.

Le logiciel Reperturb permet de comparer la distribution des longueurs CDR3 dans un échantillon donné à celle obtenue chez un individu de référence (profil gaussien, non altéré). Chaque longueur CDR3 analysée par le logiciel Immunoscope® est traduite en probabilité de distribution, en prenant en compte l'aire sous la courbe de chaque pic. Pour chacune d'entre elles, la différence de valeur absolue entre l'échantillon (En) et le profil de référence (Cn) permet d'obtenir le pourcentage d'altération de l'échantillon par rapport à un profil gaussien. Cette analyse permet donc de préciser le niveau d'altération observé sur un profil Immunoscope®. Les variations obtenues pour chaque expansion CDR3 dans une famille Vβ seront donc exprimées en pourcentage d'altération d'un pic à l'intérieur de cette famille. La somme des altérations dans toutes les familles Vβ permet d'estimer le pourcentage d'altération globale de la chaîne Vβ du TCR dans une population de cellules T.

Il faut ensuite effectuer la quantification des transcrits Vβ qui peut être abordée par plusieurs techniques. En l'espèce, on peut utiliser la méthode de la PCR quantitative en temps réel. Ce procédé, basé sur l'utilisation de la technologie TaqMan^{®}, peut utiliser le programme connu sous le nom *"ABI Prism 7700 Sequence Detection System"* qui permet entre autre de détecter et de mesurer la fluorescence émise par la liaison d'un marqueur (Sybr®Green) aux molécules d'ADN double brin. Le niveau de fluorescence, directement proportionnel à la quantité du produit dans un puits, est collecté au cours de chaque cycle d'amplification. Parallèlement aux échantillons, un standard, correspondant à une gamme de dilution de la séquence cible peut être utilisé et permettra d'établir une courbe étalon qui est utilisée pour déduire la quantité de la cible dans chaque échantillon. Afin d'éliminer les variations expérimentales entre les différents échantillons, ces valeurs sont rapportées à la mesure du gène de ménage HPRT.

Afin d'améliorer la spécificité et la sensibilité de la PCR, l'enzyme dénommée AmpliTaq Gold® DNA polymérase est utilisée. Une modification de cette enzyme la rend active uniquement à température élevée, température à laquelle l'ADN est complètement dénaturé. Afin d'éviter une contamination avec d'autres produits de PCR, l'AmpErase^{®} uracil-N-glycosylase (UNG) est ajoutée au mélange réactionnel au moment du dosage. Cette enzyme, active uniquement en dessous de 60°C, agit en hydrolysant les ponts uracil au niveau de l'ADN simple ou double brin contenant les bases uraciles et n'a aucune activité sur l'ADN contenant des thymidines. Ces ponts uracil sont générés par l'introduction des bases dUTP dans le mélange réactionnel. Lors de l'amplification, une étape initiale de 2 minutes à 50°C active cette ampérase et permet l'élimination d'éventuels contaminants issus de l'amplification précédente.

Les standards sont préparés à partir d'ARN rétrotranscrits provenant soit de splénocytes d'un animal naïf, soit de lymphocytes T issus du sang d"individus sains chez l'homme. Pour la préparation de chaque standard Vβ, l'amplification est réalisée dans un thermocycleur classique. Les produits de PCR sont ensuite déposés sur un gel d'agarose et la bande d'intérêt est extraite. La concentration du produit de PCR est estimée par la valeur DO₂₆₀ puis, en fonction de la masse moléculaire, le nombre de copies par ml est calculé. Des dilutions de chaque standard Vβ, Cβ ou HPRT à 10⁷, 10⁶, 10⁵, 10⁴, 10³ et 10² copies par puits sont ensuite préparées afin d'obtenir une gamme de concentration qui couvre celle des échantillons.

Afin de valider chaque standard, une deuxième PCR est réalisée cette fois dans *l'ABI PRISM 7700 Sequence Detector* en présence du marqueur fluorescent Sybr®Green. Les produits de PCR sont ensuite déposés sur un gel d'agarose qui permet de contrôler la diminution décroissante de chaque bande en fonction de la dilution ainsi que la taille du produit de PCR. En parallèle, l'augmentation de l'intensité de fluorescence, suivie en fonction du temps, est reliée au nombre de copies d'ADN contenues dans chaque dilution. Ceci permet de vérifier la précision des dilutions du standard, le rendement de la PCR et l'absence de contamination.

Une fois validés, ces standards permettent de déduire le nombre de copies de la cible dans chaque échantillon, testé en duplicat, en reportant le niveau de fluorescence sur la droite étalon. Cette droite n'est prise en compte que si l'efficacité de la PCR est proche de 100% (pente = -3.3) et si le coefficient de corrélation est supérieur à 0.95.

Il s'agit ensuite de combiner les analyses qualitatives et quantitatives pour donner une dimension quantitative aux altérations de la répartition des longueurs des CDR3. Cette information peut permettre d'évaluer la représentativité d'une expansion oligoclonale dans une population donnée et peut également permettre de visualiser des modifications du répertoire qui ne sont pas associées à des altérations au niveau de la région CDR3.

Un programme informatique développé afin d'obtenir une image contenant l'ensemble des informations relatives au répertoire étudié pourra être mis en oeuvre. Le logiciel de marque Matlab® peut être utilisé à cette fin puisqu'il permet ainsi la gestion souple d'un très gros volume de données et une mise en image suffisamment claire et informative. Pour chaque famille Vβ, le pourcentage d'altération par rapport au profil gaussien est mesuré par la méthode de Gorochov. Grâce au logiciel Matlab^{®}, ces valeurs sont combinées aux premières données (quantitatives) de façon à obtenir une représentation 3D illustrant les variations à la fois qualitatives et quantitatives du transcriptome Vβ dans laquelle la hauteur des pics illustre la quantité de transcrits correspondants Vβ alors que les pourcentages d'altérations sont figurés par un code couleur.

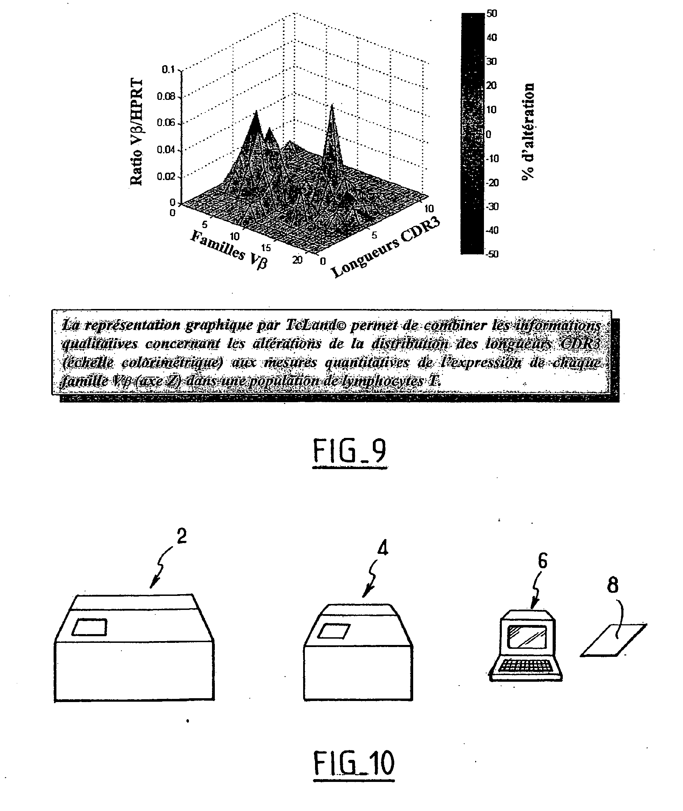

Sur le graphique de la figure 9, apparaissent :
- en abscisse, chacune des familles Vβ;
- en ordonnée, les données quantitatives (c'est à dire l'importance relative de chaque famille Vβ) ;
- selon l'axe Z, les différentes longueurs possibles du CDR3 ; et
- suivant un code de couleurs, une visualisation des altérations du répertoire par rapport à un profil gaussien.

Le code de couleurs peut comprendre une série de couleurs, par exemple du vert au rouge dans le sens de l'altération croissante, c'est à dire au fur et à mesure qu'on s'éloigne du 0% d'altération. En l'espèce, il s'agira de différents niveaux de gris, le gris étant de plus en plus foncé à mesure que l'altération augmente.

Ainsi, les transcrits Vβ altérés ou non sont exprimés selon un graphique tridimensionnel présentant en abscisse chaque famille Vβ et en ordonnée les données quantitatives obtenues par TaqMan (PCR TaqMan de Cβ et de chaque espèce de mRNA Vβ). Le pourcentage d'altération de chaque Vβ par rapport à un profil gaussien est visualisé selon un code de couleur (à chaque couleur correspond un « intervalle » d'altérations).

Le procédé sera mis en oeuvre au moyen d'une installation permettant l'analyse des récepteurs des lymphocytes T d'un organisme, illustrée schématiquement à la figure 10. Cette installation comprendra des moyens automatisés pour amplifier la région CDR3 de chacun des gènes Vβ des récepteurs des lymphocytes T associés à l'organisme (obtention des deuxièmes valeurs), et pour quantifier chaque gène Vβ (premières données). Il pourra par exemple s'agir d'une machine (2) telle que l'appareil TaqMan® qui permet, grâce à un suivi des amplifications en temps réel, de quantifier les transcrits Vβ au cours de leur amplification : les valeurs quantitatives ainsi obtenues permettent de comparer des quantités de transcrits d'une certaine famille. Vβ avec des quantités de transcrits d'une autre famille Vβ (exemple d'appareil : ABI Prism 7900 de la société Applied Biosystems).

L'installation comprendra aussi des moyens automatisés pour mesurer les quantités relatives de chaque longueur de la région CDR3 dans chaque famille Vβ et le rapport de ces quantités à des valeurs prédéterminées pour obtenir des deuxièmes données. Il pourra s'agir d'un séquenceur (4) qui permet de connaître la part relative de chaque transcrit présentant une taille particulière du CDR3 dans une famille Vβ donnée mais qui ne permet pas de comparer les familles Vβ entre elles : (par exemple le séquenceur capillaire Megabace de la société Amersham pharmacia biotech).

Enfin, l'installation comprendra des moyens automatisés pour représenter dans un diagramme à quatre variables les premières et deuxièmes données en fonction des gènes Vβ et de la longueur des régions CDR3. Il pourra s'agir d'un ordinateur (6) commandé par un programme développé par exemple à l'aide du logiciel Matlab^{®} précité. Le développement d'un tel programme en soi est à la portée de l'homme du métier et ne sera pas détaillée ici. Eventuellement, ce programme sera apte à commander d'autres étapes du déroulement du procédé, voire l'intégralité de celui-ci. Le diagramme sera représenté sur un support d'informations tel qu'un écran d'ordinateur ou un support papier (8).

Cette étude peut être réalisée au cours de la cinétique d'une réponse immune dans différentes situations.

Dans ce contexte de mise en oeuvre comme dans d'autres, l'invention permet de faire des analyses plus poussées qu'avec les méthodes de l'art antérieur. L'invention permet d'avoir une vue générale et tridimensionnelle de la taille du pool de cellules T utilisant un réarrangement Vβ particulier et permet donc de connaître l'importance quantitative des familles Vβ sélectionnées lors de la réponse immune.

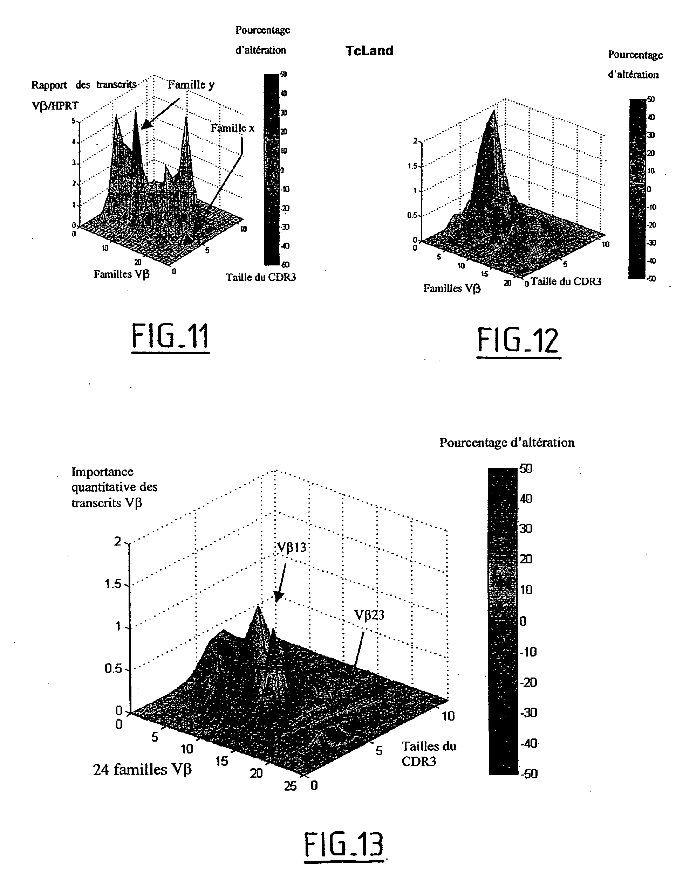

Ainsi, en référence à la figure 11 qui donne un exemple d'allure de diagramme non rattaché à des circonstances biologiques particulières, il apparaît que certaines familles, bien que fortement altérées par rapport au profil gaussien ne sont que faiblement représentées au niveau quantitatif et donc ne semblent pas avoir un rôle prépondérant (exemple : famille x). L'invention permet également de différencier une situation de repos d'une situation où les cellules T sont activées de façon polyclonale. En effet, le graphique présenté figure 12 est issu d'un modèle où des cellules T sont activées de façon polyclonale et on observe alors une très forte mobilisation de certaines familles Vβ qui, pourtant, présentent une distribution gaussienne des longueurs du CDR3 (ce qui correspond au 0% d'altération). Ainsi, cette technique permet entre autres de visualiser des activations polyclonales (ConA, superantigène, voie de présentation directe) qui n'auraient pas été détectées par les outils classiques. Il s'agit donc d'un moyen puissant pour suivre les évolutions du répertoire au cours de différentes réponses immunitaires.

En effet, le graphique est issu d'un modèle où des cellules T sont activées in vitro et on observe une très forte mobilisation de certaines familles Vβ. A cette même échelle, un échantillon témoin ou contrôle donnerait un profil vert (gris clair) car toutes les familles présentent une distribution gaussienne des longueurs du CDR3 (ce qui correspond au 0% d'altération) et plate car toutes les familles Vβ sont faiblement représentées. Il s'agit donc d'un moyen puissant pour suivre les évolutions du répertoire au cours de différentes réponses immunitaires.

On a illustré à la figure 13 le diagramme résultant d'un deuxième mode de mise en oeuvre de l'invention. L'invention est ici mise en oeuvre dans le cadre d'une étude réalisée sur des patients atteints de mélanome et vaccinés. La famille Vβ13 semble particulièrement impliquée dans la réponse anti-mélanome dans la mesure où apparaît un pic rouge (gris foncé), donc éloigné du profil gaussien caractéristique d'une réponse normale, et quantitativement important. Une autre famille semble avoir un rôle moins important : la famille Vβ23 qui est perturbée mais peu importante au niveau quantitatif. De plus, un massif apparaît au niveau des premières familles Vβ. Celui-ci pourrait correspondre à un effet de la vaccination. Des études complémentaires permettront de le déterminer.

Nous décrivons ci-après un autre mode de réalisation dans lequel le procédé de l'invention est complété par une étape d'isolement des clones présentant des familles Vβ altérées.

Les altérations spécifiques du répertoire détectées à l'aide de l'invention, ne sont pas, le plus souvent, détectables par des techniques classiques de cytométrie de flux. En effet, alors que l'invention permet de détecter une altération et une accumulation de transcrits pour une famille Vβ donnée, la cytométrie de flux permet seulement de détecter l'expression membranaire de la protéine Vβ. Or, l'expression protéique en surface est transitoire et ne permet pas de détecter une modification de l'activité transcriptionnelle d'une cellule à un moment précis.

Comme nous l'avons vu, l'invention permet ainsi de détecter une modification de l'activité transcriptionnelle pour une famille donnée. Une fois la famille repérée, il est possible de l'extraire du reste de la population lymphocytaire afin d'en étudier les caractéristiques spécifiques (phénotype, activation...). En effet, si ces caractéristiques étaient étudiées dans la population totale, elles seraient masquées par la somme des caractéristiques de toutes les autres populations de cellules T.

Le présent mode de représentation permet donc d'identifier des populations particulières impliquées dans la réponse immunitaire étudiée. Ces cellules T, portant un TCR composé d'une famille Vβ particulière, peuvent ensuite être isolées par cytomètre de flux à l'aide d'anticorps anti-Vβ par exemple, ce qui rend possible la caractérisation des clones porteurs d'altérations spécifiques (qualitatives et/ou quantitatives) du répertoire de la chaîne β du TCR.

Après mise en oeuvre de l'invention comme exposé dans les précédents modes, y compris l'étape de réalisation du diagramme, les cellules exprimant les différentes familles Vβ caractérisées par des expansions oligoclonales et/ou de grandes quantités d'ARN messagers sont sorties par cytométrie de flux grâce à des anticorps dirigés contre les différentes familles Vβ.

Ainsi, chaque famille Vβ avant tri représente moins de 5% du répertoire Vβ. Après tri, on peut travailler sur une population donnée très pure puisque la pureté obtenue est supérieure à 98%.

Plusieurs tris successifs sont possibles et donc plusieurs clones de cellules T peuvent être isolés à partir d'un même extrait cellulaire puisque après sélection d'une population de cellules T particulière, la population restante comporte tous les autres types de cellules T. Ce même extrait cellulaire qui a permis d'isoler une première population de cellules T pourra donc également servir de base pour l'extraction d'une autre famille Vβ. Les possibilités et les apports de cette technique sont donc très importants et améliorent la technique de base de l'invention. Une fois le nombre de cellules extraites déterminé, les cellules sont utilisées pour des études phénotypiques et transcriptionnelles.

Ces études sont fondamentales puisqu'elles permettront de déterminer le rôle d'un clone lymphocytaire dans une situation donnée. Cette détermination du rôle des clones de cellules T est très important dans la mesure où si le clone identifié s'avère avoir un rôle cytotoxique (virus tels que HIV, CMV, EBV ; Maladies auto-immunes, ), il faudra l'inhiber dans le cadre de stratégies d'immunothérapies. A l'inverse, si ce clone s'avère avoir un rôle protecteur (transplantation, raitements immunosuppresseurs), il conviendra de le stimuler pour obtenir un effet bénéfique.

Pour la mise en oeuvre de cette technique, on pourra utiliser un cytométre en flux qui permet d'isoler les cellules T mises en évidence par la technologie selon l'invention, après marquage des populations de cellules T considérées avec un anticorps anti-Vβ, tel que celui commercialisé sous la dénomination « Facscalibur » par la société Beckton Dickinson.

Ainsi qu'illustré par la figure 14, l'invention se rapporte également à un procédé permettant l'identification, dans des populations de lymphocytes T particulières, de gènes, de molécules et de mécanismes impliqués dans des processus physiopathologiques.

On met en oeuvre le procédé d'analyse selon l'invention et l'on peut isoler les populations de cellules T pour lesquelles les transcrits du TCR sont fortement représentés parmi l'ensembles des transcrits de la population de cellules T totale, et comparer le phénotype de ces cellules par rapport au phénotype des cellules de la population générale, permettant ainsi de mettre en évidence les voies de réponse à des stimuli.

Bien entendu, on pourra apporter de nombreuses modifications comme par exemple, sur le diagramme, la variable représentée par des différences d'aspect local du diagramme pourra être une autre que celle correspondant aux deuxièmes données.

La différence d'aspect local pourra être illustrée autrement que par des couleurs ou des nuances de gris. Il pourra par exemple s'agir de différents types de hachures ou d'autres genres de signes.

L'invention se rapporte également à chaque amorce pour l'amplification des régions CDR3, telles qu'indiquées dans la liste de séquences.

## Revendications

1. Procédé d'analyse de l'activation et de la mobilisation des cellules T par l'intermédiaire d'une analyse des récepteurs des lymphocytes T d'un organisme, **caractérisé en ce qu'**il comprend les étapes consistant à :
- déterminer le nombre de transcrits pour chaque gène Vβ, pour obtenir des premières données
- amplifier la région CDR3 des récepteurs des lymphocytes T pour chacun des gènes Vβ associés à l'organisme
- séparer les différentes longueurs du CDR3 des récepteurs des lymphocytes T pour chacun des gènes Vβ associés à l'organisme et estimer la proportion des transcrits de chaque longueur du CDR3 dans chaque famille Vβ pour obtenir des deuxièmes données
- représenter dans un diagramme à quatre variables les premières et deuxièmes données en fonction des gènes Vβ et de la longueur des régions CDR3.

2. Procédé selon la revendication précédente, **caractérisé en ce qu'**au moins une des variables est représentée sans être associée à une dimension de l'espace.

3. Procédé selon la revendication 2, **caractérisé en ce que** la variable qui n'est pas associée à une dimension de l'espace correspond aux deuxièmes données.

4. Procédé selon l'une quelconque des revendications précédentes, **caractérisé en ce qu'**au moins une des variables est représentée par des différences d'aspect local du diagramme.

5. Procédé selon la revendication 4, **caractérisé en ce que** les différences d'aspect local sont des différences de couleur.

6. Procédé selon l'une quelconque des revendications précédentes, **caractérisé en ce que** les premières données sont représentées suivant la direction verticale.

7. Procédé selon l'une quelconque des revendications précédentes, **caractérisé en ce qu'**il comprend, après la détermination des premières et des deuxièmes données, les étapes consistant à choisir un gène Vβ en fonction des résultats des deux premières étapes, et à extraire de la population de cellules T totale les cellules T portant un TCR constitué par cette famille Vβ préalablement choisie.

8. Procédé selon l'une quelconque des revendications précédentes, **caractérisé en ce qu'**au moins certaines des étapes sont mises en oeuvre de façon automatisée.

9. Installation d'analyse des récepteurs des lymphocytes T d'un organisme, **caractérisée en ce qu'**elle comprend:
- des moyens automatisés pour amplifier la région CDR3 des récepteurs des lymphocytes T pour chacun des gènes Vβ associés à l'organisme ;
- des moyens automatisés pour mesurer les quantités de chaque gène Vβ pour obtenir des premières données ;
- des moyens automatisés pour mesurer, pour chaque gène Vβ, la quantité relative de chaque région CDR3 par rapport à la totalité des régions CDR3 associées à un gène Vβ pour obtenir des deuxièmes données ; et
- des moyens automatisés pour représenter dans un diagramme à quatre variables les premières et deuxièmes données en fonction des gènes Vβ et de la longueur des régions CDR3.

## Claims

1. Method for analyzing the activation and mobilization of T cells through an analysis of the T lymphocyte receptors of an organism, **characterized in that** it comprises the steps consisting in:
- determining the number of transcripts for each Vβ gene, so as to obtain first data;
- amplifying the CDR3 region of the T lymphocyte receptors for each of the Vβ genes associated with the organism;
- separating the various lengths of the CDR3 of the T lymphocyte receptors for each of the Vβ genes associated with the organism and estimating the proportion of transcripts for each length of the CDR3 in each Vβ family, so as to obtain second data;
- representing, in a four-variable diagram, the first and second data as a function of the Vβ genes and of the length of the CDR3 regions.

2. Method according to the preceding claim, **characterized in that** at least one of the variables is represented without being associated with a dimension in space.

3. Method according to Claim 2, **characterized in that** the variable which is not associated with a dimension in space corresponds to the second data.

4. Method according to any one of the preceding claims, **characterized in that** at least one of the variables is represented by differences in local appearance of the diagram.

5. Method according to Claim 4, **characterized in that** the differences in local appearance are color differences.

6. Method according to any one of the preceding claims, **characterized in that** the first data are represented in the vertical direction.

7. Method according to any one of the preceding claims, **characterized in that** it comprises, after determination of the first and second data, the steps consisting in choosing a Vβ gene as a function of the results from the first two steps, and in extracting from the total T cell population the T cells bearing a TCR consisting of this preselected Vβ family.

8. Method according to any one of the preceding claims, **characterized in that** at least some of the steps are carried out in an automated fashion.

9. Device for analyzing the T lymphocyte receptors of an organism, **characterized in that** it comprises:
- automated means for amplifying the CDR3 region of the T lymphocyte receptors for each of the Vβ genes associated with the organism;
- automated means for measuring the amounts of each Vβ gene, so as to obtain first data;
- automated means for measuring, for each Vβ gene, the relative amount of each CDR3 region with respect to all the CDR3 regions associated with a Vβ gene, so as to obtain second data; and
- automated means for representing, in a four-variable diagram, the first and second data as a function of the Vβ genes and of the length of the CDR3 regions.

## Patentansprüche

1. Verfahren zur Analyse der Aktivierung und der Mobilisierung von T-Zellen über eine Analyse der Rezeptoren der T-Lymphozyten eines Organismus, **dadurch gekennzeichnet, dass** es die Schritte umfasst, welche daraus bestehen:
- die Anzahl von Transkripten für jedes Vβ-Gen zu bestimmen, um erste Daten zu erhalten,
- die CDR3-Region der Rezeptoren der T-Lymphozyten für jedes der Vβ-Gene, die mit dem Organismus assoziiert sind, zu amplifizieren,
- die verschiedenen Längen der CDR3 der Rezeptoren der T-Lymphozyten für jedes der mit dem Organismus assoziierten Vβ-Gene aufzutrennen und den Anteil der Transkripte von jeder Länge der CDR3 in jeder Vβ-Familie abzuschätzen, um zweite Daten zu erhalten,
- in einem Diagramm mit vier Variablen die ersten und zweiten Daten in Abhängigkeit von den Vβ-Genen und der Länge der CDR3-Regionen darzustellen.

2. Verfahren nach dem vorangegangenen Anspruch, **dadurch gekennzeichnet, dass** wenigstens eine der Variablen dargestellt wird, ohne mit einer Dimension des Raums assoziiert zu sein.

3. Verfahren nach Anspruch 2, **dadurch gekennzeichnet, dass** die Variable, die nicht mit einer Dimension des Raums assoziiert ist, den zweiten Daten entspricht.

4. Verfahren nach einem der vorangegangenen Ansprüche, **dadurch gekennzeichnet, dass** wenigstens eine der Variablen durch Unterschiede des örtlichen Aussehens des Diagramms dargestellt wird.

5. Verfahren nach Anspruch 4, **dadurch gekennzeichnet, dass** die Unterschiede des örtlichen Aussehens Farbunterschiede sind.

6. Verfahren nach einem der vorangegangenen Ansprüche, **dadurch gekennzeichnet, dass** die ersten Daten entlang der vertikalen Richtung dargestellt werden.

7. Verfahren nach einem der vorangegangenen Ansprüche, **dadurch gekennzeichnet, dass** es nach der Bestimmung der ersten und der zweiten Daten die Schritte umfasst, welche daraus bestehen, ein Vβ-Gen abhängig von den Ergebnissen der beiden ersten Schritte zu wählen und aus der gesamten Population von T-Zellen die T-Zellen zu extrahieren, welche einen TCR tragen, welcher durch diese zuvor ausgewählte Vβ-Familie gebildet wird.

8. Verfahren nach einem der vorangegangenen Ansprüche, **dadurch gekennzeichnet, dass** wenigstens bestimmte der Schritte auf automatisierte Weise ausgeführt werden.

9. Einrichtung zur Analyse der Rezeptoren der T-Lymphozyten eines Organismus, **dadurch gekennzeichnet, dass** diese umfasst:
- automatisierte Mittel, um die CDR3-Region der Rezeptoren der T-Lymphozyten für jedes der Vβ-Gene, die mit dem Organismus assoziiert sind, zu amplifizieren;
- automatisierte Mittel, um die Mengen von jedem Vβ-Gen zu messen, um erste Daten zu erhalten;
- automatisierte Mittel, um für jedes Vβ-Gen die relative Menge von jeder CDR3-Region bezogen auf die Gesamtheit der mit einem Vβ-Gen assoziierten CDR3-Regionen zu messen, um zweite Daten zu erhalten; und
- automatisierte Mittel, um in einem Diagramm mit vier Variablen die ersten und zweiten Daten in Abhängigkeit von den Vβ-Genen und der Länge der CDR3-Regionen darzustellen.
